# EUROPEAN PATENT APPLICATION

(11) **EP 1 329 195 A1**
(43) Date of publication of application: **23.07.2003**
(21) Application number: 03001173.8
(22) Date of filing: 21.01.2003
(51) Int. Cl.: A61B 17/11, A61B 17/02

(54) **An endoscopic kit**

(30) Priority: 22.01.2002 SE 0200193
(71) Applicant: JOMED NV, 6235 NS Ulestraten (NL)
(72) Inventor: Österlind, Jörgen, 263 54 Höganäs (SE)
(74) Representative: Giver, Sören Bo

(57) **Abstract**

An endoscopic kit comprises an insertion aid device having an insertion end to be inserted into said vessel and a connecting portion at an opposite end; and an endoscopic instrument with a distal end provided with a locking mechanism. The locking mechanism has a first state of operation in which the locking mechanism loosely engages the connecting portion of the insertion aid device, and a second state of operation in which the locking mechanism fixedly engages the connecting portion of the insertion aid device for locking the position of the insertion aid device in relation to the endoscopic instrument.

The endoscopic kit may be used for aiding insertion of a graft connector into a body vessel through an incisition in a wall thereof.

## Description

### Technical Field of the Invention

The present invention relates to an endoscopic kit, an endoscopic instrument, an insertion aid device and a method for aiding insertion of a graft connector into a body vessel through an incision in a wall thereof.

### Background of the Invention

Generally, endoscopic surgery is preferred to open surgery, since endoscopic surgery is less invasive. Especially, if a heart is to be treated, open surgery would require spreading the ribs apart so that access to the heart is enabled. On the other hand, endoscopic surgery is much more difficult, since access is only available through a few holes into the body. Also, movement of endoscopic instruments is also restricted inside the body. Since there are many advantages of endoscopic surgery compared to open surgery, the endoscopic surgery is being developped so that its usage could be increased.

Today, a lot of people suffer from vascular problems, such as blockings or occlusions in blood vessels. In such cases, there is a need for restoring the blood flow to parts of the body distal to the blocking. This could be accomplished by means of a graft connector, i.e. a coupling device for connecting the blood vessel distal to the blocking to a healthy blood vessel.

There are instrument for endoscopically introducing a graft connector into the blood vessel, see e.g. SE 0003606-1. However, the graft connector has a T-shape with a long toe and a shorter heel. Then, the toe is first inserted into the blood vessel through an incision in the vessel wall. The toe is pushed into the blood vessel such that the heel is fully inserted into the blood vessel, whereby a backwards retraction of the heel places the graft connector in the blood vessel extending past both sides of the incision in the blood vessel wall. However, the insertion of the heel into the blood vessel is difficult, since the combined length of the toe and the heel of the graft connector is longer than the incision made in the blood vessel wall. This is especially difficult in endoscopic surgery and particularly if the graft connector is to be coupled to a coronary vessel of a beating heart.

### Summary of the Invention

It is an object of the invention to diminish the above-mentioned problems. It is a further object of the invention to provide an aid for insertion of the heel of a graft connector.

These and other objects are achieved by an endoscopic kit according to claim 1, an endoscopic instrument according to claim 14, an insertion aid device according to claim 15, and a method according to claim 19.

Thus, according to an aspect of the invention an endoscopic kit is provided comprising an insertion aid device having an insertion end to be inserted into said vessel and a connecting portion at an opposite end; and an endoscopic instrument with a distal end provided with a locking mechanism. The locking mechanism has a first state of operation in which the locking mechanism loosely engages the connecting portion of the insertion aid device, and a second state of operation in which the locking mechanism fixedly engages the connecting portion of the insertion aid device for locking the position of the insertion aid device in relation to the endoscopic instrument.

By means of the insertion aid device being loosely engaged to the locking mechanism of the endoscopic instrument, the orientation of the insertion aid device may be altered in relation to the endoscopic instrument. Thus, the position of the insertion aid device may be adjusted for fascilitating introduction of the insertion aid device into the body vessel. The introduction of the insertion aid device into the body vessel may then be controlled freely such that the insertion aid device may be used for helping the graft connector into the body vessel.

In a preferred embodiment, the insertion aid device may comprise a hook-shaped member. Then, the hook-shaped member could be used for pushing a short side of the incision such that the opening in the vessel wall is temporarily increased. The hook-shaped member may also easily be rotated out of the body vessel.

The connecting portion of the insertion aid device may be ball shaped for providing an adjustable ball joint with said locking mechanism in said first state of operation. Such a ball joint would provide an essentially free rotation of the insertion aid device relative to the endoscopic instrument. The dimensions of an instrument for endoscopic insertion into the body is restricted by a trocar, i.e. a tubular device providing a passage into the body. Thus, the dimensions of the trocar restricts the dimensions of the endoscopic instrument. A ball joint may enable a rotation of the insertion aid device such that the insertion end projects outside the dimensions needed during the introduction of the instrument. As a result, the insertion end may be freely orientated in relation to the body vessel, which will facilitate introduction into the body vessel. Moreover, the insertion aid device may comprise a ball-shaped connecting portion connected to a hook-shaped member. This enables a hook-shaped member to be freely oriented in relation to the endoscopic instrument.

The hook-shaped member may comprise an essentially straight tip portion. Then, the tip portion will not penetrate the inside wall of the body vessel when the hook-shaped member is introduced into the vessel.

The locking mechanism may comprise two jaws for engaging the connecting portion of the insertion aid device. These jaws may engage the connecting portion on both sides, holding the connecting portion between them.

Further, at least one of the jaws may have a cup-shaped recess for engaging the connecting portion of the insertion aid device. Then, the connecting portion may not fall out of the grip of the jaws, since the distance between the jaws at edges of the recesses may be smaller than a largest dimension of the connecting portion.

A ball-shaped connecting portion of the insertion aid device may be rotated within the recesses when the locking mechanism is in the first state of operation. This may provide essentially free rotation of the insertion aid device in relation to the endoscopic instrument when the lockimg mechanism is in its first state of operation, i.e. the insertion aid device may be rotated around at least two mutually perpendicular axes.

Preferably, the dimension of the insertion aid device is such that, the endoscopic instrument and the insertion aid device engaged by said locking mechanism are introducible as a unit through an opening into the body with a cross section of 5 mm. When an endoscopic surgery is performed, a few openings into the body are formed. All instruments introduced into the body pass these openings during the surgery. Thus, the dimension of the insertion aid device is suitably within the dimensions of normal openings of the body. As a result, there is no need for a special opening for introduction of the insertion aid device.

The locking mechanism may further have a third state of operation, wherein the insertion aid device is connectable and disconnectable to the endoscopic instrument. This implies that the insertion aid device may be stored in a sterile environment until it is to be used. Then, it may be connected to the endoscopic instrument by placing it in the locking mechanism in its third state of operation and bringing the locking mechanism to its first state of operation.

Further, the endoscopic instrument may have manoeuver means for allowing a user to selectively control the state of operation of the locking mechanism from outside the body. Then, the locking mechanism may easily be brought from its first to its second state of operation when the insertion aid device has been oriented in relation to the endoscopic instrument inside the body. As a result, the insertion aid device may be locked in an orientation suitable for aiding the insertion of the graft connector.

The endoscopic kit may further comprise a positioning instrument for altering the position of the insertion aid device in relation to the endoscopic instrument, when the insertion aid device has been inserted into the body by the endoscopic instrument and is loosely engaged by the locking mechanism in its first state of operation. By affecting the insertion aid device when it is engaged by the locking mechanism in its first state of operation, the orientation of the insertion aid device may be changed. A positioning instrument is suitable for affecting the insertion aid device. The positioning instrument may be another endoscopic instrument.

The positioning instrument may have a distal end provided with gripping means for gripping the insertion aid device loosely engaged by the locking mechanism in its first state of operation. As a result, the change of orientation of the insertion aid device is simplified. By gripping the insertion aid device with the gripping means, the positioning instrument may change the orientation of the insertion aid device by dragging it to the desired orientation.

According to another aspect of the invention, an endoscopic instrument is provided, said endoscopic instrument being provided with a hook-shaped member in a distal end, wherein the orientation of said hook-shaped member in relation to the endoscopic instrument is adjustable inside the body and, thereafter, fixable in an adjusted orientation.

The hook-shaped member may be oriented in a desired orientation in relation to the endoscopic instrument. When the hook-shaped member is fixed in a desired orientation, the endoscopic instrument may affect the hook-shaped member for aiding insertion of the graft connector into the body vessel. The hook-shaped member may be used for facilitating insertion of an end of the graft connector in the end of the incision by a protruding portion of the hook-shaped member extending in the direction of the vessel away from the incision.

According to a further aspect of the invention, an insertion aid device is provided comprising a hook-shaped member having an insertion end to be inserted into said vessel, and a ball-shaped connecting portion, said connecting portion being arranged for selectively loose and fixed engagement with a distal end of an endoscopic instrument.

The insertion aid device may be connected to an endoscopic instrument for manoeuvring the insertion aid device from outside the body. Since the insertion aid device may be loosely engaged with a distal end of the endoscopic instrument, it may be suitably oriented in relation to the endoscopic instrument for aiding the insertion of the graft connector.

The insertion aid device may be made of a plastic material. Thus, it may be easily manufactured to low costs.

According to yet another aspect of the invention, there is provided a method for aiding insertion of a graft connector into a body vessel through an incision in a wall thereof, said method comprising the steps of: providing an insertion aid device having a hook-shaped insertion end to be inserted into said vessel and a connecting portion at an opposite end, connecting the connecting portion of the insertion aid device to a locking mechanism in a distal end of an endoscopic instrument, said locking mechanism having a first state of operation in which the locking mechanism loosely engages the connecting portion of the insertion aid device, and a second state of operation in which the locking mechanism fixedly engages the connecting portion of the insertion aid device for locking the orientation of the insertion aid device in relation to the endoscopic instrument, locating the insertion aid device at said incision in the vessel wall by means of the endoscopic instrument, re-orientating the insertion end of the insertion aid device in relation to the endoscopic instrument while said locking mechanism is in its first state of operation, and thereafter bringing the locking mechanism to its second state of operation in order to lock orientation of said insertion aid device in relation to the endoscopic instrument.

Thus, insertion of a graft connector into a body vessel may be simplified. The heel end of the graft connector may be guided by the insertion aid device into the body vessel. When the orientation of the insertion aid device is locked in relation to the endoscopic instrument, the endoscopic instrument may be used for manoeuvring the insertion aid device. Thus, the steps of re-orientating the insertion aid device and bringing the locking mechanism to its second state of operation may place the insertion aid device in a proper position in relation to the endoscopic instrument for further manoeuvring of the insertion aid device by means of the endoscopic instrument.

### Brief Description of the Drawings

The invention will now be described in more detail by way of example referring to the appended drawings.
Fig. 1 is a bottom view of an insertion aid device.
Fig. 2 is a front view of the insertion aid device.
Fig. 3 is a side view of the insertion aid device.
Fig. 4 is a cross sectional view of the insertion aid device along line IV-IV in Fig. 3.
Fig. 5 is a cross sectional side view of the insertion aid device.
Fig. 6 is a side view of an endoscopic instrument.
Fig. 7 is a side view of the endoscopic instrument when engaging the insertion aid device.
Fig. 8 is a cross sectional view of a jaw of a locking mechanism of the endoscopic instrument along line VIII-VIII in Fig. 6.
Fig. 9 is a cross sectional view of a jaw of a locking mechanism of the endoscopic instrument along line IX-IX in Fig. 6.
Fig. 10 is a bottom view of the upper jaw of the locking mechanism of the endoscopic instrument.
Fig. 11 is a schematic view of an introduction of the endoscopic instrument with the insertion aid device into the body.
Fig. 12 is a schematic view of re-orientation of the insertion aid device in relation to the endoscopic instrument.
Fig. 13 is a schematic view of a correctly orientated insertion aid device.
Fig. 14 is a schematic view of introduction of the insertion aid device into a body vessel.
Fig. 15 is a schematic view of introduction of a graft connector into the body vessel.

### Detailed Description of a Preferred Embodiment of the Invention

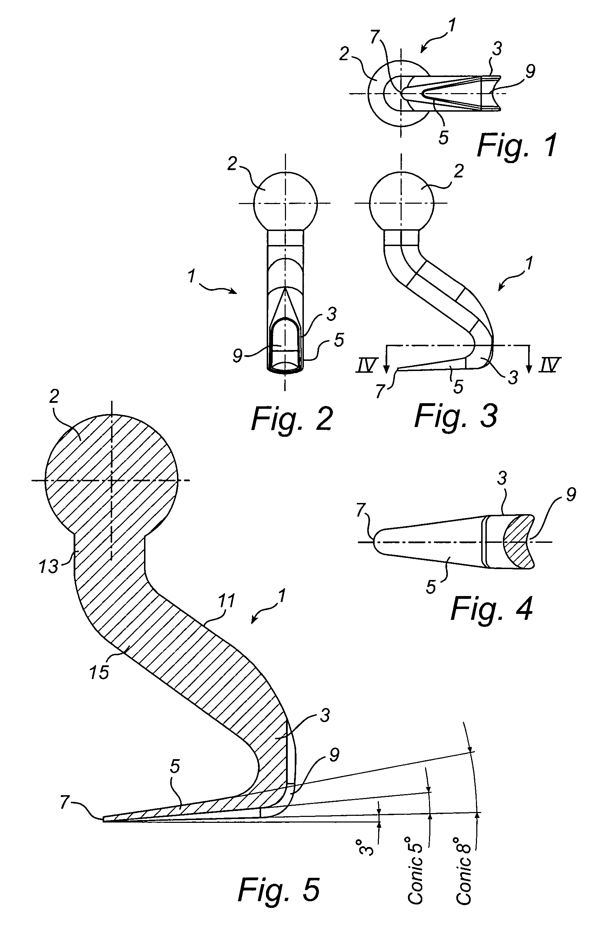

Referring to Figs 1-5, an insertion aid device 1 according to an embodiment of the invention will now be described. The insertion aid device 1 comprises an insertion end 3. The insertion end 3 is to be inserted into a body vessel for aiding insertion of a graft connector into the body vessel. The insertion aid device further comprises a connecting portion 2 in an opposite end of the device. The connecting portion 2 is to be connected to an endoscopic instrument for controlling the insertion aid device when inserted into a body. The connecting portion 2 comprises a ball shape for engagement with the endoscopic instrument.

The insertion end 3 forms a hook having a tip arm 5, which extends in an essentially straight direction. The tip arm 5 tapers towards a tip 7. The insertion end 3 is also provided with a groove 9, extending along a surface of the insertion aid device from the insertion end 3 towards the connecting portion 2. The groove 9 extends along the tip arm 5 and the outside of an angled portion of the hook.

The connecting portion 2 and the insertion end 3 are connected by an arm 11. The arm 11 extends in a first portion 13 in a first direction from the connecting portion 2. The arm 11 is angled into a second portion 15 extending in a second direction towards the insertion end 3. The angles of the arm 11 and the hook are in opposite directions. Thereby, the insertion aid device 1 may be inserted through an opening of a small diameter without rotating the insertion aid device 1, while having a longer length of the arm 11 than otherwise possible. A longer arm 11 may make the insertion aid device 1 more easily handled.

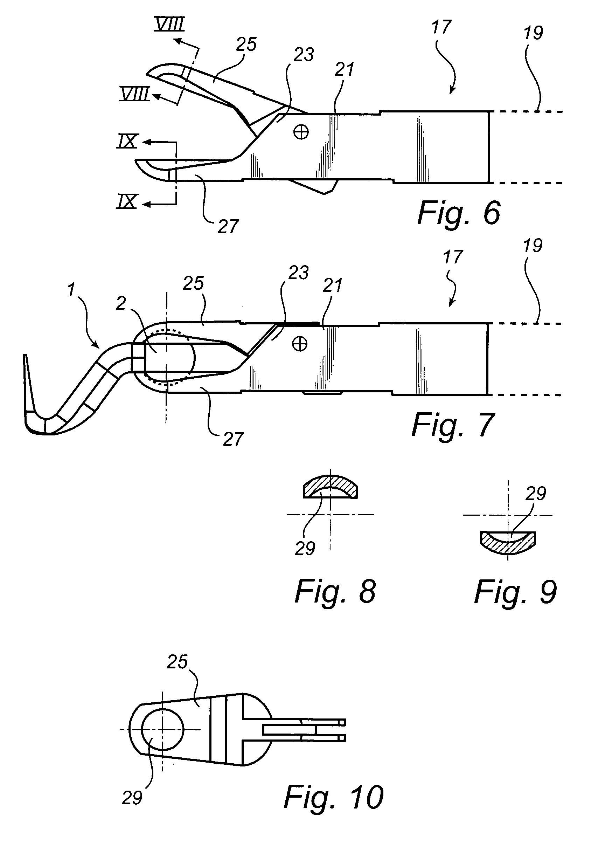

Referring now to Figs 6-10, an endoscopic instrument 17 according to an embodiment of the invention will be described. The endoscopic instrument 17 comprises a shaft 19 having a distal end 21 and a proximal end. In the distal end 21 of the shaft 19, there is provided a locking mechanism 23. In the proximal end, the shaft 19 is connected to a manoeuvring means 25 for controlling the locking mechanism 23.

The locking mechanism 23 is arranged for engaging the connecting portion 2 of the insertion aid device 1. The locking mechanism 23 comprises a pair of jaws 25, 27. The jaws 25, 27 are arranged for engaging the connecting portion 2 between them as shown in Fig. 7. The jaws 25, 27 are moveable in relation to each other. In one embodiment, the upper jaw 25 is hingedly connected to the lower jaw 27 such that upon movement back and forth of a control rod extending along the longitudinal direction of the shaft 19 by means of the manoeuvring means, the upper jaw 25 is rotated away from or towards the lower jaw 27.

Each jaw 25, 27 has a cup-shaped recess 29, as best seen in Figs 8 and 9. The recess is formed for taking up the ball-shaped connecting portion 2 of the insertion aid device 1. When the locking mechanism 23 engages the connecting portion 2 of the insertion aid device 1, the ball-shaped connecting portion 2 will abut the recesses 29. Thus, the connecting portion 2 is positioned in secure engagement with the locking mechanism 23. In this position, the jaws 25, 27 are too close to each other at the edges of the recesses 29 for allowing the connecting portion 2 to be released from the engagement with the locking mechanism. However, this is a loose engagement, since the connecting portion 2 forms a ball joint with the cup-shaped recesses 29. The insertion aid device 1 may then be rotated essentially freely in relation to the endoscopic instrument 17.

The engagement of the locking mechanism 23 with the connecting portion 2 may be locked in a specific orientation by pressing the upper jaw 27 towards the lower jaw 25 and locking the movement of the upper jaw 27 by means of the manoeuvring means. Then, the orientation of the insertion aid device 1 in relation to the endoscopic instrument 17 is locked.

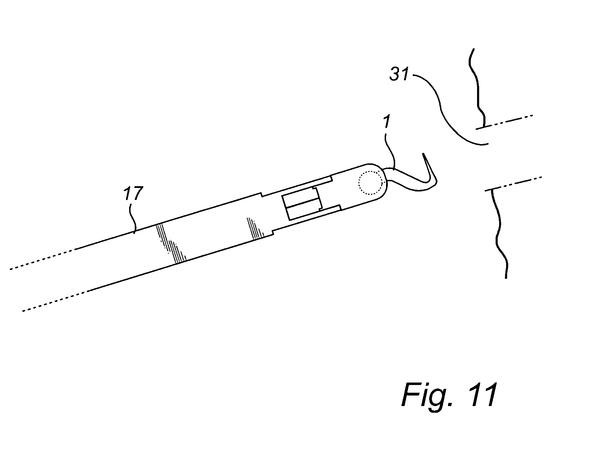

Referring to Figs 11-15, the use of the insertion aid device for aiding insertion of a graft connector into a body vessel through an incision in a vessel wall thereof will be described. Referring to Fig. 11, the insertion aid device 1 is introduced into the body in a position in relation to the endoscopic instrument such that the insertion aid device 1 has a minimal extension in a direction perpendicular to the longitudinal axis of the endoscopic instrument 17. Thus, the insertion aid device 1 may be inserted through a normal opening 31 into the body used in endoscopic surgery. The endoscopic instrument 17 is then pushed to locate the insertion aid device at the incision in the vessel wall.

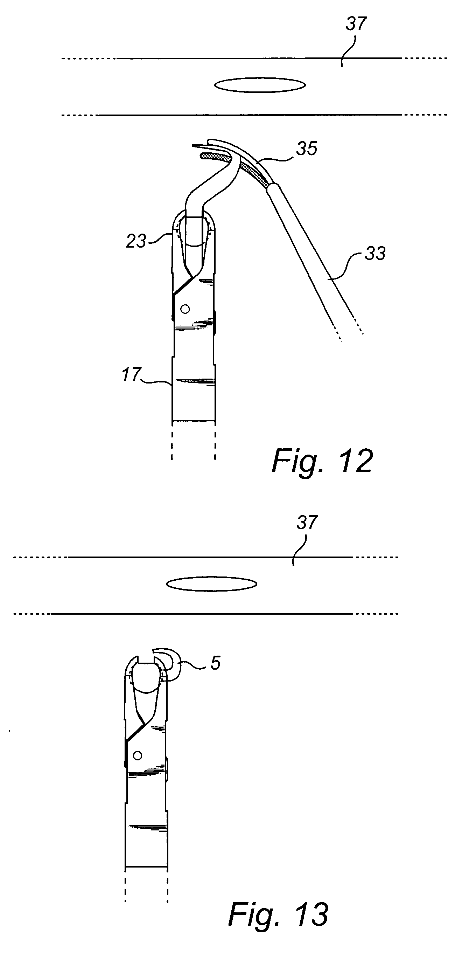

Then, the locking mechanism 23 of the endoscopic instrument 17 is brought into loose engagement with the insertion aid device 1. Thus, the insertion aid device 1 may be orientated in relation to the endoscopic instrument 17. The change of orientation of the insertion aid device 1 is made to adjusting the direction of the insertion aid device 1 in relation to the body vessel. The change of orientation may be accomplished by inserting a distal end of another endoscopic instrument, hereafter called positioning instrument 33, to the position of the insertion aid device 1, as shown in Fig. 12. The positioning instrument 33 is introduced into the body through an opening other than the opening used for the endoscopic instrument 17. The positioning instrument 33 may be a commonly known endoscopic instrument having a pair of forceps 35 in the distal end. The pair of forceps 35 may be used for gripping the insertion aid device 1 in the arm 11 or the insertion end 3. Then, the insertion aid device 1 may be rotated in relation to the endoscopic instrument 17 around the connecting portion 2 of the insertion aid device 1. The rotation may be accomplished by moving the positioning instrument 33, thereby guiding the insertion aid device 1 along the movement of the positioning instrument 33. The insertion aid device 1 may be orientated such that the tip arm 5 extends in parallel with the body vessel 37, as shown in Fig. 13. When the insertion aid device 1 has been properly orientated, the grip of the positioning instrument 33 holding the insertion aid device 1 is released and the positioning instrument 33 is withdrawn.

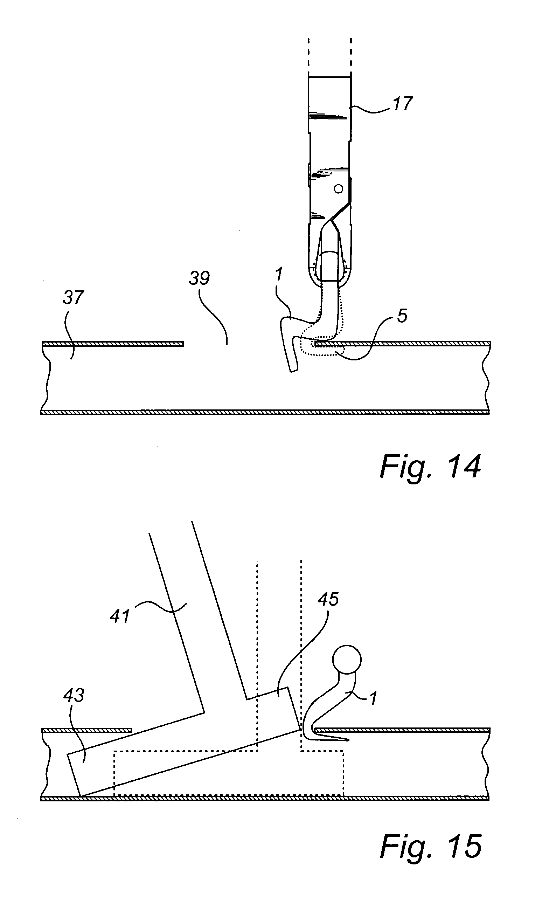

When the insertion aid device 1 has been properly orientated, the locking mechanism 23 of the endoscopic instrument 17 is brought into locked engagement with the connecting portion 2 of the insertion aid device 1. Then, a rotation of the shaft 19 of the endoscopic instrument 17 around its longitudinal axis may rotate the tip 7 of the insertion aid device 1 with respect to the incision 39 in the vessel wall. Then, the insertion aid device 1 is guided by the endoscopic instrument 17 to the end of the incision. There, the insertion aid device 1 is rotated by rotation of the shaft 19 of the endoscopic instrument 17 such that the insertion aid device 1 is introduced into the vessel, as shown in Fig. 14. The insertion aid device 1 is introduced such that the tip arm 5 extends along the longitudinal axis of the vessel directed away from the incision 39 in the vessel wall. The tapering shape of the tip arm 5 is suitable for simple introduction of the tip arm 5 into the vessel.

The hook-shaped insertion end 3 may be used for extending the size of the incision 39 by pulling the incision edge in a direction along the vessel. Now, the graft connector 41 may be easily introduced into the vessel. The graft connector 41 may be introduced into the body through the same opening used for the positioning instrument 33. When the graft connector has been brought to the body vessel, the toe portion 43 of the graft connector 41 is first introduced by the end of the incision opposite the end where the insertion aid device 1 is introduced, as shown by solid lines in Fig. 15. Then, the heel portion 45 may be easily guided into the vessel along the surface of the insertion aid device 1. The end of the heel may engage the groove 9 of the insertion aid device 1 and be guided by the groove 9 into the body vessel. When the graft connector 41 is positioned inside the body vessel, as shown by dashed lines in Fig. 15, the insertion aid device 1 may be rotated out of the vessel by rotating the shaft 19 of the endoscopic instrument 17.

## Claims

1. An endoscopic kit for aiding the insertion of a graft connector into a body vessel through an incision in the wall thereof, said kit comprising:
an insertion aid device having an insertion end to be inserted into said vessel and a connecting portion at an opposite end; and
an endoscopic instrument with a distal end provided with a locking mechanism, said locking mechanism having (i) a first state of operation in which the locking mechanism loosely engages the connecting portion of the insertion aid device, and (ii) a second state of operation in which the locking mechanism fixedly engages the connecting portion of the insertion aid device for locking the position of the insertion aid device in relation to the endoscopic instrument.

2. The endoscopic kit according to claim 1, wherein the insertion aid device comprises a hook-shaped member.

3. The endoscopic kit according to claim 1 or 2, wherein the connecting portion of the insertion aid device is ball shaped for providing an adjustable ball joint with said locking mechanism in said first state of operation.

4. The endoscopic kit according to claim 1-3, wherein the insertion aid device comprises a ball-shaped connecting portion connected to a hook-shaped member.

5. The endoscopic kit according to any one of claims 2-4, wherein the hook-shaped member comprises an essentially straight tip portion.

6. The endoscopic kit according to any one of claims 1-5, wherein the locking mechanism comprises two jaws for engaging the connecting portion of the insertion aid device.

7. The endoscopic kit according to claim 6, wherein at least one of said jaws has a cup-shaped recess for engaging the connecting portion of the insertion aid device.

8. The endoscopic kit according to any one of claims 1-7, wherein, in said first state of operation of the locking mechanism, the connecting portion of the insertion aid device is essentially freely rotatable in relation to the endoscopic instrument.

9. The endoscopic kit according to any one of claims 1-8, wherein the dimension of the insertion aid device is such that, the endoscopic instrument and the insertion aid device engaged by said locking mechanism are introducible as a unit through an opening into the body with a cross section of 5 mm.

10. The endoscopic kit according to any one of claims 1-9, wherein the locking mechanism further has a third state of operation, wherein the insertion aid device is connectable and disconnectable to the endoscopic instrument.

11. The endoscopic kit according to any one of claims 1-10, wherein the endoscopic instrument has manouver means for allowing a user to selectively control the state of operation of the locking mechanism from outside the body.

12. The endoscopic kit according to any one of claims 1-11, further comprising a positioning instrument for altering the position of the insertion aid device in relation to the endoscopic instrument, when the insertion aid device has been inserted into the body by the endoscopic instrument and is loosely engaged by the locking mechanism in its first state of operation.

13. The endoscopic kit according to claim 12, wherein the positioning instrument has a distal end provided with gripping means for gripping the insertion aid device loosely engaged by the locking mechanism in its first state of operation.

14. An endoscopic instrument for aiding insertion of a graft connector into a body vessel through an incision in the wall thereof, said endoscopic instrument being provided with a hook-shaped member in a distal end, wherein the orientation of said hook-shaped member in relation to the endoscopic instrument is adjustable inside the body and, thereafter, fixable in an adjusted orientation.

15. An insertion aid device for aiding endoscopical insertion of a graft connector into a blood vessel through an incision in a wall thereof, the insertion aid device comprising
a hook-shaped member comprising an insertion end to be inserted into said vessel, and
a ball-shaped connecting portion, said connecting portion being arranged for selectively loose and fixed engagement with a distal end of an endoscopic instrument.

16. The insertion aid device according to claim 15, wherein the insertion end comprises an essentially straight tip portion.

17. The insertion aid device according to claim 15 or 16, wherein the dimension of the insertion aid device is such that, when being fixedly engaged to the endoscopic instrument, it is introducible through an opening into the body with a cross section of 5 mm.

18. The insertion aid device according to any one of claims 15-17, wherein the insertion aid device is made of a plastic material.

19. A method for aiding insertion of a graft connector into a body vessel through an incision in a wall thereof, said method comprising the steps of:
providing an insertion aid device having a hook-shaped insertion end to be inserted into said vessel and a connecting portion at an opposite end,
connecting the connecting portion of the insertion aid device to a locking mechanism in a distal end of an endoscopic instrument, said locking mechanism having a first state of operation in which the locking mechanism loosely engages the connecting portion of the insertion aid device, and a second state of operation in which the locking mechanism fixedly engages the connecting portion of the insertion aid device for locking the orientation of the insertion aid device in relation to the endoscopic instrument,
locating the insertion aid device at said incision in the vessel wall by means of the endoscopic instrument,
re-orientating the insertion end of the insertion aid device in relation to the endoscopic instrument while said locking mechanism is in its first state of operation, and
thereafter bringing the locking mechanism to its second state of operation in order to lock orientation of said insertion aid device in relation to the endoscopic instrument.
